# EUROPEAN PATENT APPLICATION

(11) **EP 1 566 184 A1**
(43) Date of publication of application: **24.08.2005**
(21) Application number: 03758790.4
(22) Date of filing: 22.10.2003
(51) Int. Cl.: A61K 45/00, A61P 25/00, A61P 27/02, A61P 43/00, A61K 38/22

(54) **REMEDY FOR CORNEAL FAILURE**

(30) Priority: 31.10.2002 JP 2002318881; 18.02.2003 JP 2003040250
(71) Applicant: Senju Pharmaceutical Co., Ltd., Osaka 541-0046 (JP)
(72) Inventor: TAKAYAMA, Yoshiko, Kobe-shi, Hyogo 655-0003 (JP); NAKAMURA, Yoshikuni, Kobe-shi, Hyogo 653-0841 (JP); INOUE, Jun, Kobe-shi, Hyogo 654-0103 (JP); AZUMA, Mitsuyoshi, Nishinomiya-shi, Hyogo 662-0031 (JP)
(74) Representative: Helbing, Jörg
(86) International application number: PCT/JP2003/013503
(87) International publication number: WO 2004/039403

(57) **Abstract**

The present invention provides a new type of pharmaceutical agent that recovers corneal sensitivity after corneal surgery or improves the condition of dry eye. Application of a somatostatin receptor agonist is expected to provide an improvement effect on decreased corneal sensitivity after cataract surgery or LASIK surgery, decreased corneal sensitivity and dry eye associated with corneal neurodegeneration such as neuroparalytic keratopathy, corneal ulcer, diabetic keratopathy and the like.

## Description

### Technical Field

The present invention relates to a corneal nerve axon extension promoter comprising a somatostatin receptor agonist, and an agent for the recovery or improvement of corneal sensitivity or the treatment of dry eye or corneal epithelial defect, based on the extension of corneal nerve axon.

### Background Art

Since corneal nerve is severed by corneal surgeries such as Laser photorefractive keratectomy (PRK), Laser-Assisted-In-Situ Keratomileusis (LASIK), keratoplasy and the like, the corneal sensitivity is said to decrease generally for about 3 weeks to one year. As a result of the functional decrease of corneal sensitivity, patients after a corneal surgery blink less number of times, problematically showing the symptoms of dry eye. In the patients with dry eye, lacrimal hypofunction gives rise to corneal hyposensitivity, which, upon combination with further lacrimal hypofunction, problematically aggravates the condition of corneal surface. At present, however, recovery of corneal sensitivity after a corneal surgery is left to spontaneous recovery, and in the treatment of dry eye, no active treatment is provided to recover corneal sensitivity.

Somatostatin is a peptide isolated from hypothalamus in 1973 as a somatotropin release inhibiting factor (SRIF), and five subtypes of somatostatin receptors have been found to the present day, which are respectively named SSTR1, SSTR2, SSTR3, SSTR4 and SSTR5. Somatostatin is widely distributed as a somatotropin release inhibitory factor in the nerve tissues, and the presence of somatostatin receptor in the eye tissue has been confirmed for iris, ciliary body and retina (Mori, M. et al., Neuroscience Letters, 1997, vol. 223, No. 3, pp. 185-188).

In addition, somatostatin has a great diversity of functions in the endocrine system, exocrine system, nerve system and the like in living organisms, and has been reported to be involved in, for example, neurotransmission, nerve cell growth regulation and the like, and has a promoting action on the nerve axon extension in PC12 cells (Ferriero, M.D. et., Developmental Brain Research, vol. 80, p. 13-18 (1994)) .

As the eye diseases involving somatostatin, glaucoma, inflammation of corneal stroma, iritis, retinitis, cataract, conjunctivitis and the like are known (JP-A-2002-515912, corresponding to WO98/58646, EP1019050).

In addition, an attempt has been made to develop somatostatin itself or a derivative thereof as a pharmaceutical product and, for example, octreotide known as a somatostatin receptor agonist is commercially available as a therapeutic drug for gastrointestinal tract hormone producing tumor and acromegaly ? pituitary gigantism. In addition, for example, the following are known: lanreotide (JP-A-2-289599, corresponding to EP389180), AN-238 (JP-A-2000-502055, corresponding to WO97/19954, US5843903), PTR-3173 (JP-A-2002-518339, corresponding to US6051554, US6355613), an amine derivative having affinity for SSTR2 or SSTR3 (JP-A-2000-226373, corresponding to US6329389), an aromatic amine derivative having a somatostatin receptor function regulating action and useful for the prophylaxis or treatment of diabetes, obesity, diabetic complications and the like (JP-A-2000-191615, corresponding to EP1123918), a fused ring compound having a somatostatin receptor agonist action and useful for the prophylaxis or treatment of diabetes and the like (JP-A-11-209356, corresponding to US6352982),
for example, peptide having a somatostatin-like activity, which is represented by formula I

X¹-X²-Asn-Phe-Phe-Trp-Lys-Thr-Phe-X³-Ser-X⁴

wherein X¹ is Asp-Arg-Met-Pro-Cys, Arg-Met-Pro-Cys, Met-Pro-Cys, Pro-Cys or Cys, X² is Arg or Lys, X³ is Ser or Thr, and X⁴ is Cys-Lys or Cys (JP-A-10-174587),
for example, a somatostatin agonist represented by formula II 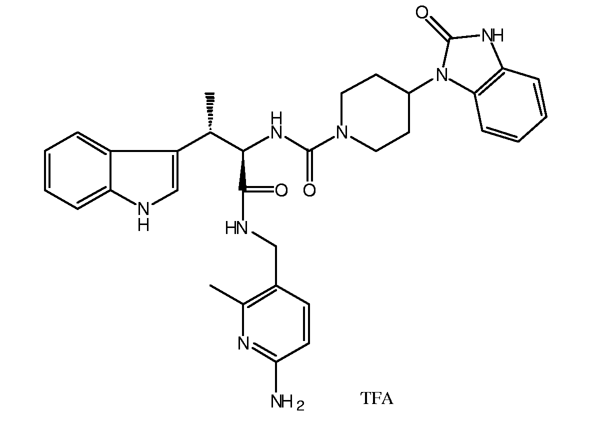 and the like (JP-A-2001-518895, corresponding to WO98/45285, EP977751),
for example, a somatostatin agonist represented by formula III 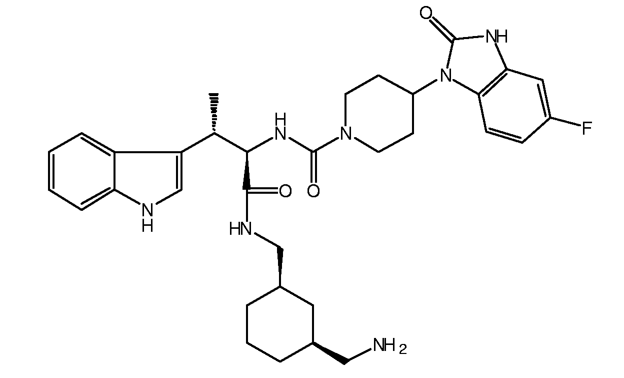 and the like (JP-A-2001-519811, corresponding to WO98/44921, US6063796),
for example, a somatostatin agonist represented by formula IV 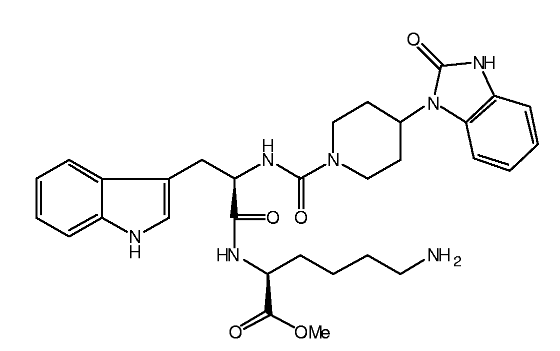 and the like (JP-A-2001-519812, corresponding to WO98/44922, US6063796),
a somatostatin agonist represented by formula V 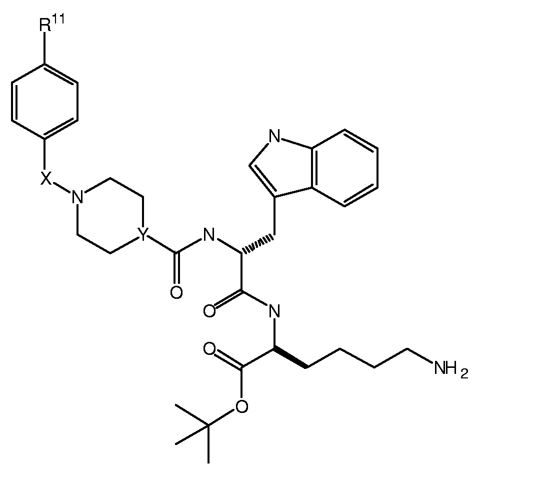 wherein R¹¹ is a group selected from a halogen atom, a cyano group, a carboxy group, a (C₁-C₆)alkyl group and a (C₁-C₆)alkoxy group; X is a -CH₂- group, a -SO₂- group, a -CO- group or a direct bond; and Y is a CH group or a nitrogen atom, and the like (JP-A-2001-114761, corresponding to EP1086947A1),
for example, a somatostatin agonist represented by formula VI 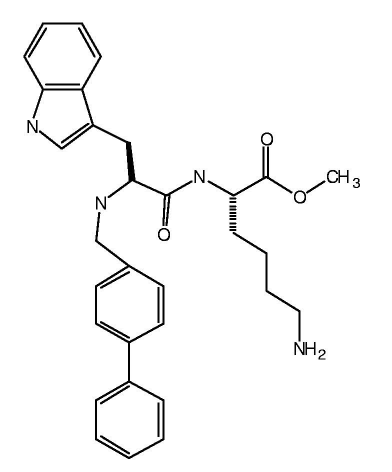 and the like (JP-A-2002-3498, corresponding to US2001/047030), for example, a somatostatin agonist acting on SSTR2, such as 5-guanidino-2((2-(toluene-4-sulfonyl)-1,2,3,4-tetrahydroisoquinoline-3-carbonyl)-amino)-pentanoic acid methyl ester and the like (US2002/91125A1),
for example, a somatostatin agonist represented by formula VII 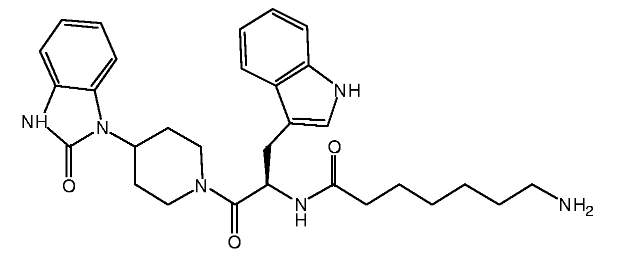 and the like (US2002/91090A1),
a somatostatin analog represented by formula VIII 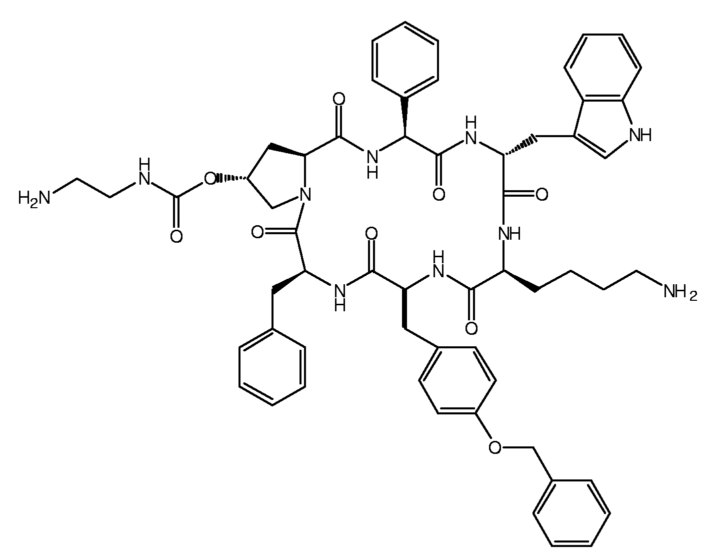 (WO2002/10192),
an imidazolyl derivative inducing an agonist effect from one or more subtype receptors of somatostatin (WO99/64401), a hydantoin derivative having affinity for somatostatin receptor (WO2001/85718), a 4-aminopiperidine derivative useful as a somatostatin receptor ligand (WO2001/44191),
for example, a somatostatin agonist represented by formula IX 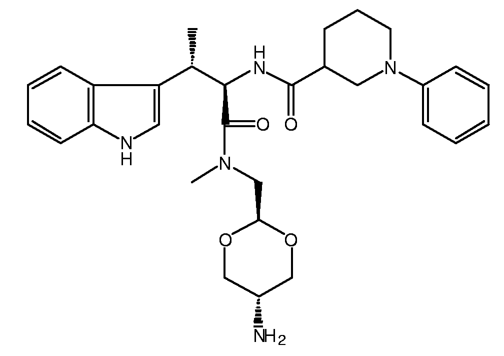 and the like (US6387932),
for example, an SSTR1 agonist represented by Phe-cyclo(Cys-D-Trp-Lys-Cys)-Thr-NH₂ (WO2000/75186),
a compound represented by formula X 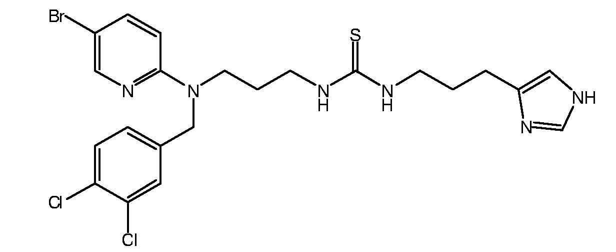 and the like, having a selective SSTR4 binding action and expected to have a glaucoma treatment action (US6127343),
a somatostatin analog represented by formula XI 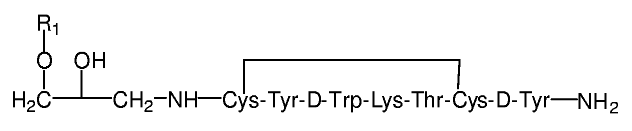 wherein R₁ is C₁-C₄ alkyl, adamantyl and the like (WO02000/10589) for example, a somatostatin agonist represented by formula XII 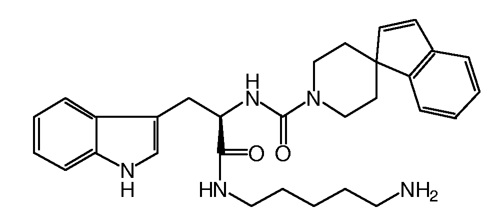 and the like (WO99/22735, corresponding to US6117880), for example, a somatostatin agonist represented by formula XIII 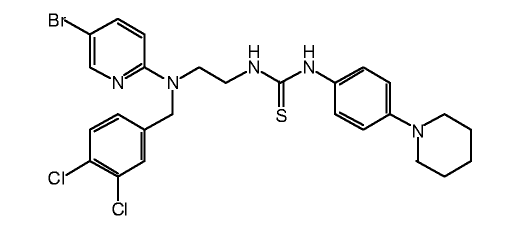 and the like (JP-A-2001-502712, corresponding to US6020349, US6083960),
for example, a somatostatin agonist represented by formula XIV 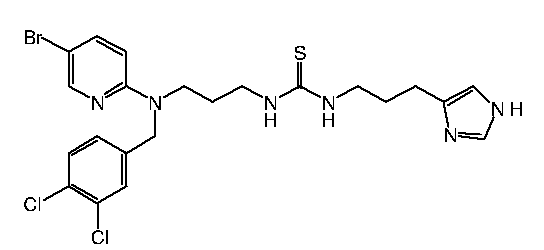 and the like (JP-A-2001-525793 (corresponding to WO97/43278, EP912551), for example, a cyclic somatostatin analog represented by cyclo[Tyr-D-Trp-Lys-Val-Phe(4-(3-methoxyphenyl)imidazole)-Gly] and the like (JP-A-2002-518409 (corresponding to WO99/65942, EP1086131), an ergoline derivative, which is an SSTR1 selective agonist useful for the treatment of anxiety, depression and the like (JP-A-2001-527580 (corresponding to WO98/54183, US6221870), a biphenyl compound as a somatostatin receptor function regulator (JP-A-2002-80439, corresponding to W02002/000606), a β-carboline derivative that binds with a somatostatin receptor to block Na channel (JP-A-2002-517500, corresponding to WO99/64420, EP1086101), vapreotide (Sharon Gazal et al., Journal of Medicinal Chemistry, vol. 45, p. 1665-1671 (2002)), an amine derivative which is a compound having a somatostatin receptor binding-inhibitory action and having a specific chemical structure characterized by the substitution of the 2-position of a group: 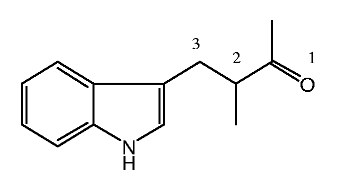 with a nitrogen atom (JP-A-2002-348287), pyridothienodiazepin having affinity and selectivity for a somatostatin receptor (JP-A-2002-541260, corresponding to W02000/61587) and the like.

In contrast, there is no report on the presence of a somatostatin receptor in cornea. With regard to the corneal nerve, moreover, a report has documented that most of the nerves derived from the first branch (ophthalmic branch) separating from the trigeminal ganglia are distributed in the cornea, and deeply involved in the recovery of sensitivity after corneal surgery, regeneration of corneal epithelium and the like (Ke-Ping Xu et al. Cornea, vol. 15, pp. 235-239 (1996)).

However, no report has been found that teaches the presence of somatostatin receptor in the trigeminal nerve (corneal nerve) or promotion of nerve axon extension of trigeminal nerve (corneal nerve) by somatostatin.

### Disclosure of the Invention

The present invention provides a pharmaceutical agent that shows functional recovery of corneal sensitivity in patients having functional decrease of corneal sensitivity, from patients after corneal surgery such as Laser photorefractive keratectomy (PRK), Laser-In-Situ Keratomileusis (LASIK), keratoplasy and the like, patients with dry eye and the like, and that treats disorders of corneal epithelium associated with these functional decrease of corneal sensitivity.

The present inventors have studied for the purpose of providing a new type of pharmaceutical agent that recovers corneal sensitivity after corneal surgery or improves the condition of dry eye and first found that somatostatin has an axon extension promoting effect for the trigeminal nerve (hereinafter sometimes to be referred to as corneal nerve), and that a somatostatin receptor is present in the trigeminal nerve. They have further studied based on these findings, and completed the present invention that utilizes a somatostatin receptor agonist as a drug for the recovery of corneal sensitivity and the like.

Accordingly, the present invention relates to
(1) a corneal nerve axon extension promoter comprising a somatostatin receptor agonist,
(2) an agent for the recovery of corneal sensitivity, which comprises a somatostatin receptor agonist,
(3) a therapeutic agent for dry eye, which comprises a somatostatin receptor agonist,
(4) a therapeutic agent for corneal epithelial defect, which comprises a somatostatin receptor agonist,
(5) a pharmaceutical composition for promoting extension of corneal nerve axon, which comprises a somatostatin receptor agonist,
(6) a pharmaceutical composition for the recovery of corneal sensitivity, which comprises a somatostatin receptor agonist,
(7) a pharmaceutical composition for the treatment of dry eye, which comprises a somatostatin receptor agonist,
(8) a pharmaceutical composition for the treatment of corneal epithelial defect, which comprises a somatostatin receptor agonist,
(9) use of a somatostatin receptor agonist for the production of a pharmaceutical composition for promoting extension of corneal nerve axon,
(10) use of a somatostatin receptor agonist for the production of a pharmaceutical composition for the recovery of corneal sensitivity,
(11) use of a somatostatin receptor agonist for the production of a pharmaceutical composition for the treatment of dry eye,
(12) use of a somatostatin receptor agonist for the production of a pharmaceutical composition for the treatment of corneal epithelial defect,
(13) a method of promoting extension of corneal nerve axon, which comprises administering an effective amount of a somatostatin receptor agonist to a case in need of the promotion of extension of the corneal nerve axon,
(14) a method of recovering corneal sensitivity, which comprises administering an effective amount of a somatostatin receptor agonist to a case in need of the recovery of corneal sensitivity,
(15) a method of treating dry eye, which comprises administering an effective amount of a somatostatin receptor agonist to a case affected with dry eye, and
(16) a method of treating corneal epithelium defect, which comprises administering an effective amount of a somatostatin receptor agonist to a case having corneal epithelial defect.

As used herein, by the somatostatin receptor agonist is meant somatostatin *per se,* as well as those that act on somatostatin receptor and show an action similar to that of somatostatin, and encompasses those referred to as somatostatin agonist, somatostatin analogous form, somatostatin analog and the like.

As the somatostatin receptor agonist, somatostatin per se, as well as any compound can be advantageously used as long as it acts on a somatostatin receptor and shows an action similar to that of somatostatin. As such compound, for example, octreotide known as a somatostatin receptor agonist, lanreotide described in JP-A-2-289599 (corresponding to EP389180), octapeptides such as vapreotide and the like, somatostatin-like cyclic peptides described in JP-A-2000-502055 (corresponding to WO97/19954, US5843903), such as AN-238 and the like, main chain cyclized somatostatin analog described in JP-A-2002-518339 (corresponding to US6051554, US6355613) such as PTR-3173 and the like, amine derivatives disclosed in JP-A-226373 (corresponding to US6329389) and JP-A-2002-348287, aromatic amine derivatives described in JP-A-2000-191615 (corresponding to EP1123918), fused ring compounds described in JP-A-11-209356 (corresponding to US6352982), peptides described in JP-A-10-174587, somatostatin agonists described in JP-A-2001-518895 (corresponding to WO98/45285, EP977751), JP-A-2001-519811 (corresponding to WO98/44921, US6063796) and JP-A-2001-519812 (corresponding to WO98/44922, US6063796), somatostatin agonists described in JP-A-2001-114761 (corresponding to EP1086947A1), JP-A-2002-3498 (corresponding to US2001/047030), US2002/91125A1, US2002/91090A1, US6387932, WO99/22735 (corresponding to US6117880) and JP-A-2001-502712 (corresponding to US6020349, US6083960), somatostatin analogs described in W02002/10192 and W02000/10589, imidazolyl derivatives described in WO99/64401, hydantoin derivatives described in W02001/85718, 4-aminopiperidine derivatives described in W02001/44191, SSTR1 agonists described in W02000/75186, compounds described in US6127343, which have a selective SSTR4 binding action and a glaucoma treatment action, somatostatin agonists as described in JP-A-2001-525793 (corresponding to WO97/43278, EP912551), cyclic somatostatin analogs described in JP-A-2002-518409 (corresponding to WO99/65942, EP1086131), ergoline derivatives described in JP-A-2001-527580 (corresponding to WO98/54183, US6221870), biphenyl compounds described in JP-A-2002-80439 (corresponding to W02002/000606), β-carbolyl derivatives described in JP-A-2002-517500 (corresponding to WO99/64420, EP1086101), pyridothienodiazepin described in JP-A-2002-541260 (corresponding to W02000/61587) and the like can be mentioned.

A pharmaceutical agent containing the somatostatin receptor agonist of the present invention is useful for the recovery from functional decrease of corneal sensitivity of cornea of mammals (e.g., human, rat, mouse, rabbit, bovine, pig, dog, cat and the like), wherein the corneal nerve is damaged or cut or the corneal epithelium is defective. For example, it is useful as a therapeutic drug for the recovery of decreased corneal sensitivity after surgery such as PRK, LASIK, keratoplasty and the like, or as a therapeutic drug for dry eye patients with decreased corneal sensitivity, and further as a therapeutic drug for a corneal epithelial disorder associated with functional degradation of corneal sensitivity. As the somatostatin receptor agonist, an agonist that specifically acts on SSTR2 and/or SSTR4, which are somatostatin receptor subtypes, is more preferable.

The pharmaceutical agent of the present invention is systemically or topically administered. Systemically, it is orally administered, and parenterally, it is administered as intravenous injection, subcutaneous injection, intramuscular injection and the like. Topically, it is administered to the eye.

As the dosage form of the pharmaceutical agent of the present invention, solid agents such as powder, granule, tablet, capsule, suppository and the like; liquids such as syrup, injection, eye drop and the like; and the like can be mentioned. For the production of granules and tablets, any dosage form can be produced by using, for example, excipients (lactose, sucrose, glucose, starch, microcrystalline cellulose and the like), lubricants (magnesium stearate, talc, stearic acid, calcium stearate and the like), disintegrants (starch, carmellose sodium, calcium carbonate and the like), binders (starch paste solution, hydroxypropylcellulose solution, carmellose solution, gum arabic solution, gelatin solution, sodium alginate solution and the like) and the like. For granules and tablets, a coating film may be formed using suitable coating agents (gelatin, sucrose, gum arabic, carnauba wax and the like), enteric coatings (e.g., cellulose acetate phthalate, metacrylic acid copolymer, hydroxypropylcellulose phthalate, carboxymethylethylcellulose and the like) and the like.

For the production of capsule, a mixture of suitable excipients such as magnesium stearate, calcium stearate, talc, light silicic anhydride and the like for improving flowability and glidability, microcrystalline cellulose, lactose and the like for flowability under pressurization, as well as the above-mentioned disintegrant and the like added as appropriate is uniformly admixed or granulated or granulated, coated with a suitable coating agent to form a film and packed in a capsule, or encapsulation-molded with a capsule base having increased plasticity, which contains a suitable capsule base (gelatin and the like), glycerin or sorbitol and the like. These capsules may contain coloring agents, preservatives [sulfur dioxide, parabens (methyl paraoxybenzoate, ethyl paraoxybenzoate or propyl paraoxybenzoate)] and the like as necessary. The capsule may be a conventional one, an enteric coated capsule, a gastric coated capsule or a release control capsule. When an enteric capsule is produced, a compound coated with an enteric coated agent or the above-mentioned suitable excipients are added to a compound and packed in a conventional capsule or a capsule itself may be coated with an enteric coating agent, or an enteric polymer may be used as a base for molding.

For the production of a suppository, a base for suppository (e.g., cacao butter, macrogol and the like) can be appropriately selected and used.

For the production of syrup, for example, stabilizers (sodium edetate and the like), suspending agents (gum arabic, carmellose and the like), corrigents (simple syrup, glucose and the like), aromatic and the like can be appropriately selected and used.

For the production of the pharmaceutical agent of the present invention as an injection or eye drop, it can be produced by dissolving or dispersing the agonist in a solution appropriately containing pharmaceutically acceptable additives such as isotonicity agents (sodium chloride, potassium chloride, glycerin, mannitol, sorbitol, boric acid, borax, glucose, propylene glycol and the like), buffers (phosphate buffer, acetate buffer, borate buffer, carbonate buffer, citrate buffer, Tris buffer, glutamate buffer, e-aminocaproate buffer and the like), preservatives (p-oxybenzoic acid esters, chlorobutanol, benzyl alcohol, benzalkonium chloride, sodium dehydroacetate, sodium edetate, boric acid, borax and the like), thickeners (hydroxyethyl cellulose, hydroxypropyl cellulose, polyvinyl alcohol, polyethylene glycol and the like), stabilizers (sodium bisulfite, sodium thiosulfate, sodium edetate, sodium citrate, ascorbic acid, dibutylhydroxytoluene and the like), pH adjusting agents (hydrochloric acid, sodium hydroxide, phosphoric acid, acetic acid and the like), and the like.

While the amount of the additives to be used for the above-mentioned syrup, injection and eye drop varies depending on the kind of the additives to be used, use and the like, they may be added at a concentration capable of achieving the purpose of the additive, and an isotonicity agent is generally added in about 0.5 - about 5.0 w/v% to make the osmotic pressure about 229 - about 343 mOsm. In addition, a buffer is added in about 0.01 - about 2.0 w/v%, a thickener is added in about 0.01 - about 1.0 w/v%, and a stabilizer is added in about 0.001 - about 1.0 w/v%. A pH adjusting agent is appropriately added to generally achieve a pH of about 3 - about 9, preferably about 4 - about 8.

For particular use as an eye drop, the lower limit of the concentration of the somatostatin receptor agonist is adjusted to generally about 0.0005 w/v%, about 0.001 w/v% or about 0.005 w/v% and the upper limit is adjusted to about 1.0 w/v%, about 0.5 w/v%, about 0.1 w/v%, about 0.05 w/v% or about 0.01 w/v%.

While the dose of the somatostatin receptor agonist of the present invention varies depending on the target disease, symptom, subject of administration, administration method and the like, when, for example, it is topically administered to the eye of an adult after PRK surgery as an agent for the recovery of corneal sensitivity, for example, a liquid eye drop containing about 0.01 w/v% of somatostatin is preferably instilled into the eye several times a day at about 20 to about 50 µL per dose.

When base, amino acid and the like are expressed with abbreviations in the present description and Figures, they are based on the abbreviations according to the IUPAC-IUB Commission on Biochemical Nomenclature or abbreviations conventionally used in the art. Examples thereof are shown in the following. When amino acid has an optical isomer, it is an L-isomer unless otherwise specified.
DNA: deoxyribonucleic acid
cDNA: complementary deoxyribonucleic acid
DNase: deoxyribonuclease
SSTR: somatostatin receptor
GAPDH: glyceraldehyde-3-phosphate-dehydrogenase
NGF: nerve growth factor
A: adenine
T: thymine
G: guanine
C: cytosine
RNA: ribonucleic acid
mRNA: messenger RNA
Gly: glycine
Ala: alanine
Val: valine
Ser: serine
Thr: threonine
Cys: cysteine
Met: methionine
Asp: aspartic acid
Lys: lysine
Arg: arginine
Phe: phenylalanine
Tyr: tyrosine
Trp: tryptophan
Pro: proline
Asn: asparagine
Me: methyl group

### Brief Description of the Drawings

Fig. 1 shows the expression of somatostatin receptor subtypes (SSTR2 and SSTR4) in the trigeminal nerve and retina of rabbits.
Fig. 2 shows phase contrast microscopic images of cultured rabbit trigeminal nerve cells and axon extension from the cells, wherein A shows the cells of a non-addition group, B shows the cells of a 1 µM somatostatin addition group, C shows the cells of a 10 µM somatostatin addition group, D shows the cells of an NGF addition group, and E shows the cells of a somatostatin+NGF addition group.
Fig. 3 is a graph showing the shift of the corneal sensitivity after cutting the corneal nerve of rabbits, wherein * means a significant difference from the vehicle administration group (n=6-12, mean ± standard error, p<0.05).
Fig. 4 shows fluorescence microscopic images showing cultured rabbit trigeminal nerve cells and axon extension from the cells, wherein A shows the cells of a control group and B shows the cells of a 10 µM octoreotide addition group.
Fig. 5 is a graph showing the rate (%) of neuritogenetic cells to the total cell number in the same test as Fig. 4, wherein * shows a significant difference (n=3, mean ± standard error, p<0.05) from the control.
Fig. 6 shows fluorescent microscopic images of cultured rabbit trigeminal nerve cells and axon extension from the cells, wherein A shows the cells of a non-addition group, B shows the cells of a 1 µM compound 1 addition group and C shows the cells of a 0.1 µM compound 2 addition group.
Fig. 7 is a graph showing the absorbance indicative of the amount of neurofilament of cultured cells (n=3, mean ± standard error), in the same test as Fig. 6.

### Best Mode for Carrying Out the Invention

The present invention is explained in more detail by referring to the following Experimental Examples and Examples, which are not to be construed as limitative.

### Experimental Example 1 Expression of somatostatin receptor in the trigeminal nerve of rabbit

### 1) Animals used

Japanese White Rabbit (body weight 2.0 kg) purchased from Fukusaki Rabbit Warren was used.

### 2) Test method

A mixture of Celactal (xylazine):Ketalar (ketamine hydrochloride)=0.5:1 was intramuscularly injected (0.9 mL/kg) to Japanese White Rabbit for systemic anesthesia. After cardiac perfusion with saline, retina and trigeminal ganglia were respectively obtained. By AGPC method using a TRIzol Reagent (manufactured by GIBCO BRL), RNA was extracted from each tissue, genome DNA was removed by DNase treatment, and a reverse transcription reaction was performed using SuperScripit II (manufactured by GIBCO BRL). cDNA was amplified using Platinum Taq DNA polymerase (GIBCO BRL) under the reaction conditions described in Table 1. As the primer, a rabbit somatostatin receptor SSTR2 gene specific primer (SEQ ID NO:1 mentioned later) and an SSTR4 gene specific primer (SEQ ID NO:2 mentioned later), shown in Table 1, were used. The retina and GAPDH were used as the positive control of the expression.

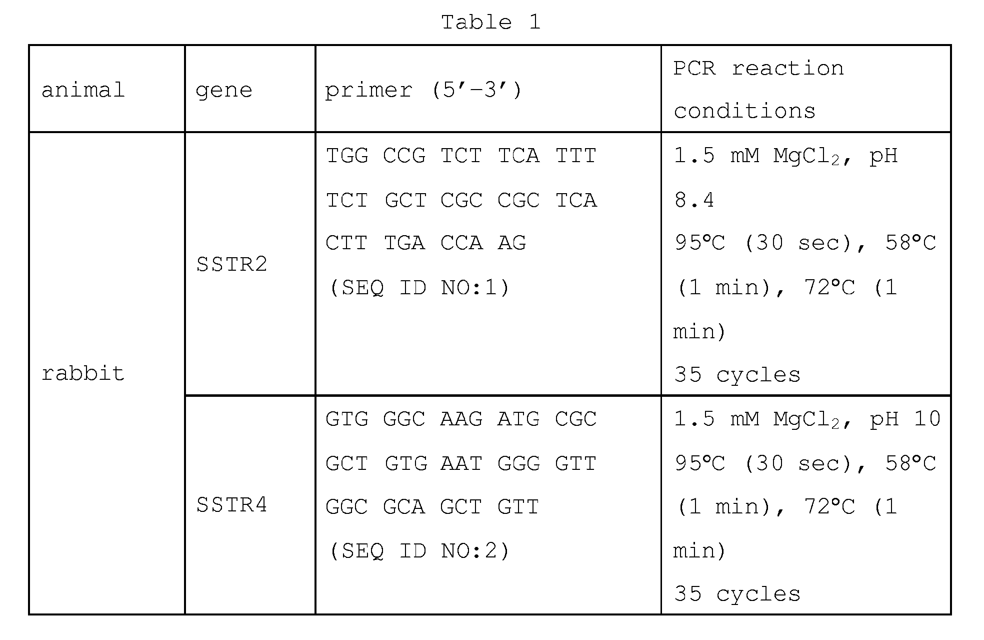

### 3) Test results

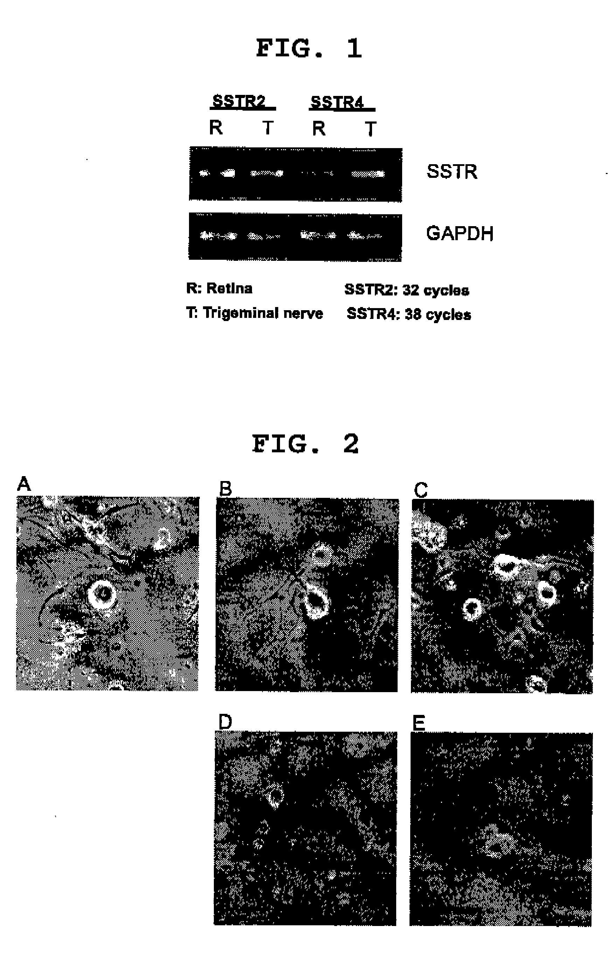

The results are shown in Fig. 1. The somatostatin receptor subtypes SSTR2 and SSTR4 in rabbit retina (R) and trigeminal nerve (T) were analyzed by RT-PCR. As a result, expression of both SSTR2 and SSTR4 was found in the both tissues.

### Experimental Example 2 Nerve axon extension promoting action in cultured rabbit trigeminal nerve cells (in vitro experiment)

### 1) Animals used

Japanese White Rabbits (2-3 days old) purchased from Fukusaki Rabbit Warren were used.

### 2) Test substance

As a test substance, somatostatin (manufactured by CALBIOCHEM, Lot B33795) and NGF (NGF-7S, manufactured by Sigma) were used. The test substance was dissolved in phosphate buffer (PBS) to 100 µM somatostatin, 20 µg/mL NGF-7S. The prepared reagents were preserved at -80°C and dissolved before use.

### 3) Test method

The trigeminal nerve cell was isolated according to the report of Chan et al. (Kuan Y. Chan and Richard H. Haschke. Exp. Eye Res. 41: 687-699, 1985). To be specific, under ether anesthesia, after cardiac perfusion of rabbit with saline, the trigeminal ganglia was removed, trigeminal ganglia was dispersed using a nerve dispersion solution (SUMITOMO BAKELITE Co., Ltd.), and the cells were plated in a 24-well plate (SUMITOMO BAKELITE Co., Ltd.) coated with polylysine/laminin. The number of cells was about 3000 cells per well and the culture conditions were 5% CO₂, 95% air at 37°C. The cells were innoculated in Dulbecco's Modified Eagle Medium/Nutrient Mixture F-12 (DMEM/F-12) culture medium supplemented with 5% fetal calf serum (FCS), cultured for 24 hr and the culture medium was exchanged to FCS-free DMEM/F-12 culture medium.

Thereafter, the cells were divided into the following 5 groups:
I non-addition group
II a group with addition of somatostatin to final concentration of 1 µM
III a group with addition of somatostatin to final concentration of 10 µM
IV a group with addition of NGF to final concentration of 1 µg/mL and
V a group with simultaneous addition of somatostatin and NGF to final concentrations of 1 µM and 1 µg/mL, respectively, and cultured for 48 hr.

The effect on the formation of neurite and the axon extension from trigeminal nerve cell by somatostatin and NGF were morphologically evaluated by the observation using a phase contrast microscope.

### 4) Test results

Fig. 2 shows phase contrast microscopic images of cultured rabbit trigeminal nerve cells, wherein (A) shows the cells of group I, (B) shows the cells of group II, (C) shows the cells of group III, (D) shows the cells of group IV, and (E) shows the cells of group V, respectively.

The formation of neurite was slightly observed in the cells of the non-addition group (A). As compared to (A), the cells (B) of 1 µM somatostatin addition group showed apparent promotion of axon extension, and in the cells (C) of the 10 µM somatostatin addition group, too, many cells showing long axon extension were observed. In the cells (D) of the NGF addition group and the cells (E) of NGF, somatostatin simultaneous addition group, too, a clear nerve cell axon extension-promoting action was observed as compared to non-addition group (A).

### Experimental Example 3 Functional changes in corneal sensitivity after rabbit corneal nerve cutting (in vivo test)

### 1) Animals used

Male Japanese White Rabbits (body weight 1.5 kg-2.0 kg) purchased from Fukusaki Rabbit Warren were used.

### 2) Test substance

As the test substance, somatostatin (manufactured by CALBIOCHEM, Lot B33795) and NGF (NGF-7S, manufactured by Sigma) were used. The test substances were dissolved in the vehicle shown below and used for the test.

| **Formulation of vehicle** | |
|---|---|
| sodium chloride | 0.9 g |
| sodium dihydrogen phosphate dihydrate | 0.1 g |
| sodium hydroxide | suitable amount (pH 7.0) |
| distilled water for injection | suitable amount |
| total amount | 100 mL |

### 3) Test method

A mixture of Celactal (xylazine):Ketalar (ketamine hydrochloride)=0.5:1 was intramuscularly injected (0.9 mL/kg) to the rabbits for systemic anesthesia. Cornea was labeled with trephine having a diameter of 6 mm, and incised at the upper side thereof for 180 degrees in a circle while suturing with a 8.0 nylon suture thread. A vehicle (vehicle administration group, n=10), a somatostatin solution (100 µM, somatostatin administration group, n=12) or an NGF solution (20 µg/mL, NGF administration group, n=6) was instilled into the eye by 50 µL 4 times/day for 4 consecutive weeks starting from immediately after operation. For a week after the operation, a tarivid ophthamic solution (Santen) was simultaneously instilled with the instillation of the test substance 4 times a day. The animals were bred in a breeding chamber set at room temperature 23±3°C, humidity 55±10%, 12 hr illumination (8:00 light on, 20:00 light off) and housed at one animal per cage. The corneal sensitivity was measured using a Cochet-Bonnet corneal sensitivity meter (manufactured by LUNEAU) at day 3 and 1, 2, 3, 4 and 6 weeks of the operation. The corneal sensitivity (%) was calculated based on the sensitivity of each individual before operation as 100%.

### 4) Test results

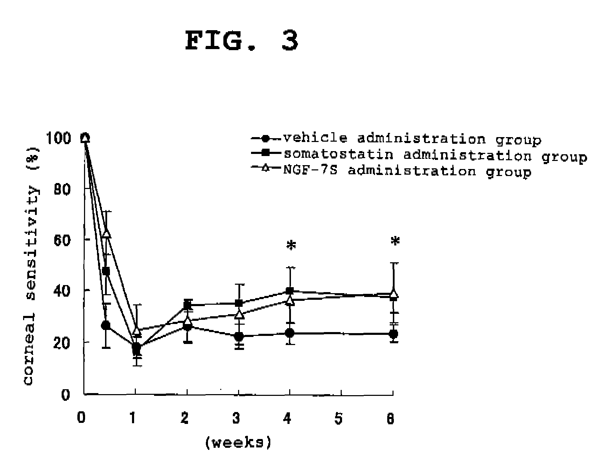

Fig. 3 shows the shift of corneal sensitivity with the lapse of time after cutting of the corneal nerve. The corneal sensitivity rapidly decreased in all groups from the third day to one week after the keratotomy, but mild recovery of the corneal sensitivity was found after 2 weeks. Three or four weeks later, the somatostatin administration group showed a significant corneal sensitivity recovery effect as compared to the vehicle administration group (p<0.05, Dunnett's test).

### Experimental Example 4 Effect of Octreotide on axon extension of cultured rabbit trigeminal nerve cells (in vitro test)

### 1) Animals used

Japanese White Rabbits (2-3 days old) purchased from Fukusaki Rabbit Warren were used.

### 2) Test substance

Octreotide (SMS 201-995, American Peptide Company, Inc.) was used.

### 3) Test method

The cell culture: Trigeminal nerve cells were isolated by reference to the report of Chan et al. (Kuan Y. Chan and Richard H. Haschke. Exp. *Eye Res.* 41: 687-699, 1985). To be specific, under ether anesthesia, after cardiac perfusion of rabbit with saline, the trigeminal ganglia was removed, and dispersed in nerve dispersion solution (SUMITOMO BAKELITE Co., Ltd.). The cells were counted and innoculated in an 8 well culture slide (BECTON DICKINSON) coated with polylysine. The cell number was about 3×10³ cells per well, and the culture conditions were 5% CO₂, 95% air, 37°C. For cell culture, Neurobasal medium (GIBGO) added with B27 supplement (GIBGO; 0.02 mL/mL culture solution) was used, and Octreotide (10 µM final concentration) was added to the medium immediately after cell inoculation and the cells were cultured for 24 hr. Immunostaining: At 24 hr from culture, the cells were fixed with 4% paraformaldehyde at room temperature for 2 hr, and nerve cell, axon and dendrite were fluorescence stained with anti-neurofilament 200 antibody (NF, Anti-Neurofilament 200, Sigma) specifically recognizing a neurofilament constituting the nerve cell and neurite. The images of the stained cells were imported into a computer from the fluorescence microscope, and the effect of Octreotide on the neurite formation and axon extension was evaluated using an image analysis soft (MacSCOP, MITANI CO.). To be specific, the axon extension length and the diameter of the cell were measured using an image analysis soft, the cells including an axon having a length of not less than twice the diameter of the cell were taken as neuritogenic cells and the percentage (%) thereof to the total cell number was calculated.

### 4) Test results

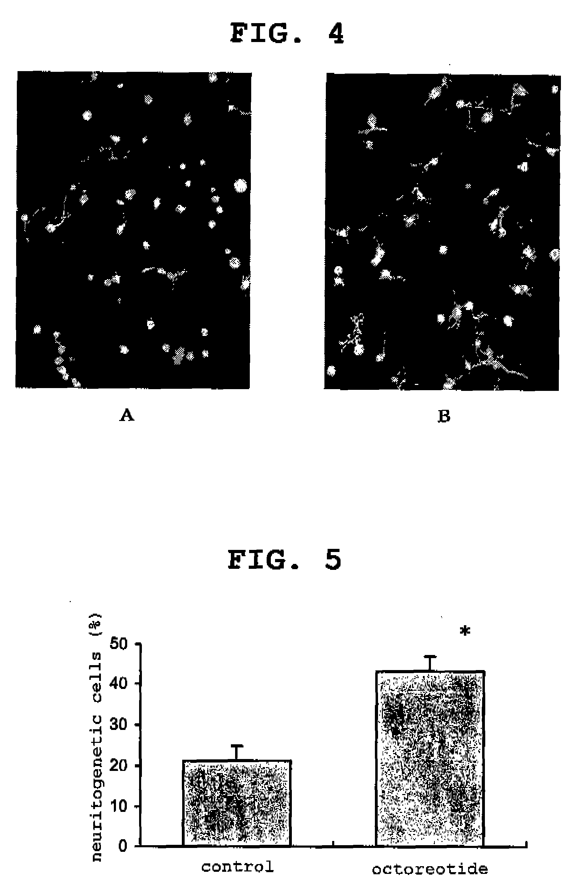

Fig. 4 shows an extension effect of Octreotide on neurite and axon in cultured rabbit trigeminal nerve cells, wherein (A) shows control rabbit trigeminal nerve cells cultured in an Octreotide-free medium for 24 hr, and (B) shows cells cultured for 24 hr in a medium containing Octreotide at a final concentration of 10 µM. It was confirmed that the neuritogenic cells increased in the Octreotide addition group as compared to the control cell group.

Fig. 5 shows a proportion (%) of neuritogenic cell to the total cell number. The proportion of the neuritogenic cells was about 21% of the total cells in the control group, about 43% of the total cells in the Octreotide addition group, and a significant increase in the neuritogenic cells due to the addition of Octreotide was observed (n=3, mean ± standard error, t-test, p<0.05%).

From the foregoing, it was found that Octreotide, which is a somatostatin analog, promotes the axon extension of trigeminal nerve cell.

### Experimental Example 5 Effect of somatostatin receptor agonist on axon extension of cultured rabbit trigeminal nerve cell (in vitro test)

### 1) Animals used

Japanese White Rabbits (2-3 days old) purchased from KITAYAMA LABES Co., Ltd. were used.

### 2) Test substance

As a somatostatin receptor agonist, which is a test substance, t-butyl 6-amino-2-(3-(1H-indol-3-yl)-2-((4-(2-oxo-2,3-dihydrobenzoimidazol-1-yl)piperidine-1-carbonyl)amino)propionylamino)hexanoate, which is an SSTR2 specific agonist (hereinafter to be referred to as compound 1), and 1-(3-(N-(5-bromopyridin-2-yl)-N-(3,4-dichlorobenzyl)amino)propyl)-3-(3-(1H-imidazol-4-yl)propyl)thiourea (hereinafter to be referred to as compound 2), which is an SSTR4 specific agonist, were used. The compound 1 was a compound of Example 4 and synthesized according to the method of Example 2 of JP-A-2001-519812 (WO98/44922). The compound 2 was synthesized according Example 15 of JP-A-2001-525793 (WO97/43278).

### 3) Test method

### Cell culture:

Rabbit trigeminal nerve cells were isolated by reference to the report of Chan et al. (Kuan Y. Chan and Richard H. Haschke. Exp. *Eye Res.* 41: 687-699, 1985). To be specific, under ether anesthesia, cardiac perfusion with saline was performed and trigeminal ganglia was removed. Using nerve dispersion solution (SUMITOMO BAKELITE Co., Ltd.), trigeminal ganglia was dispersed to prepare trigeminal nerve cells. For cell culture, Neurobasal medium (GIBCO) added with B27 Supplement (GIBCO; final concentration 2% v/v) and L-Glutamine (GIBCO; final concentration 1 mM) was used under the culture conditions of 5% CO₂, 95% air, 100% humidity, 37°C, 48 hr. The cells were innoculated on an 8 well culture slide (BECTON DICKINSON) coated with polylysine at about 3×10³ cells/well, or on a 96 well plate coated with polylysine at 3×10³ cells/well and the medium of the test substance group contained compound 1 (final concentration 1 µM) or compound 2 (final concentration 0.1 µM).
Cell staining: After the completion of 48 hr culture, the cells innoculated on a 96 well plate were fixed with 4% paraformaldehyde and the neurofilament of the cell was labeled with anti-neurofilament 200 antibody (Sigma) specifically recognizing a neurofilament constituting nerve cell body and neurite and HRP conjugated goat anti-mouse IgG antibody (Wako Pure Chemical Industries, Ltd.). Onto the labeled cells was added a citrate buffer containing 50 µL of 0.02% H₂O₂ (Nacalai Tesque) and 0.2% o-phenylenediamine (Sigma) to allow color development for 30 min. Then, 50 µL of 4.5 M H₂SO₄ (Nacalai Tesque) was added to quench the reaction. After the completion of coloring, the absorbance at 492 nm was measured and the measured value was taken as the amount of the stained neurofilament and used as an index for neuritogenesis (Taniwaki T., et al. Dev. Brain Res. (1995) 88, 109-166). The cells plated on an 8 well culture slide were fixed with 4% paraformaldehyde, a sample fixed with an anti-neurofilament 200 antibody (Sigma) was stained, and fluorescence labeled with Alexa Fluor 568 conjugated secondary antibody (Molecular probes). The fluorescence labeled cells were observed under a fluorescence microscope and the cell images were imported into a computer.

### 4) Test results

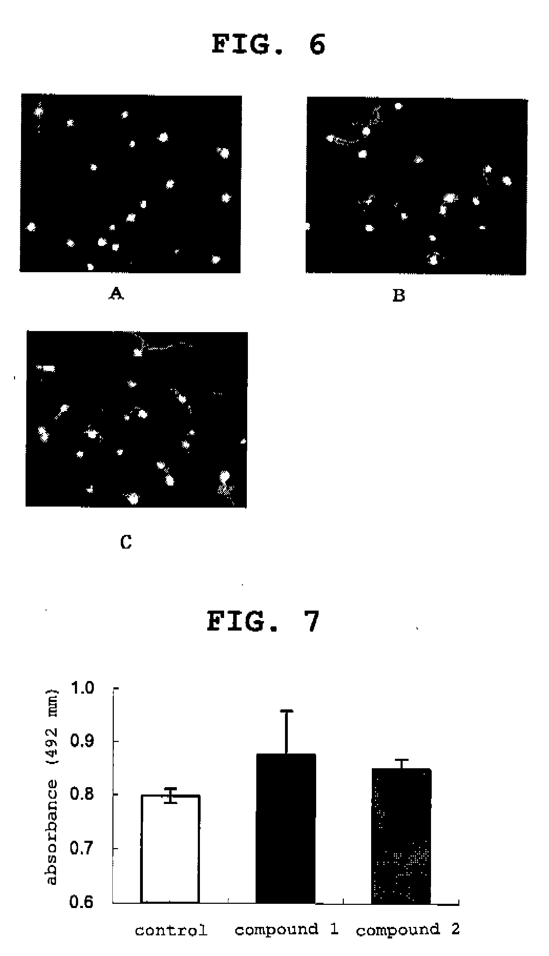

Fig. 6 shows fluorescence microscopic images of cultured rabbit trigeminal nerve cells, wherein (A) shows the cells of the control group cultured in a somatostatin receptor agonist-free medium, (B) shows the cells of the group with the addition of compound 1 at a final concentration of 1 µM, and (C) shows the cells of the group with the addition of compound 2 at a final concentration of 0.1 µM. As compared with the cells of the control group, the cells of the group with the addition of compound 1 or compound 2 were confirmed to show an increase in the neuritogenic cells. Fig. 7 is a graph showing the absorbance indicating the neurofilament amount of the cells of each addition group, wherein the absorbance of the control group was 0.798, and the absorbance of the compound 1 addition group and compound 2 addition group was 0.876 and 0.850, respectively. That is, the amount of neurofilament increased depending on the addition of the somatostatin receptor agonist, which is considered to reflect the increase in the neuritogenic cells.

From the foregoing, it was found that compound 1 and compound 2, which are somatostatin receptor agonists, have an action to promote axon extension of trigeminal nerve cell.

Formulation Examples are shown in the following.

### Example 1: Tablet

| | |
|---|---|
| somatostatin | 50 mg |
| lactose | 80 mg |
| starch | 17 mg |
| magnesium stearate | 3 mg |
| Microcrystalline cellulose | 10 mg |

Using the above components as a material for one tablet, tablets are molded according to a conventional method. The tablets may be coated as necessary with a conventional enteric coating (e.g., hydroxypropylmethylcellulose phthalate and the like), or a sugar coating or film (e.g., ethylcellulose).

### Example 2: capsule

| | |
|---|---|
| somatostatin | 75 mg |
| mannitol | 75 mg |
| starch | 17 mg |
| calcium stearate | 3 mg |

Using the above components as a material for one capsule, they are uniformly mixed, granulated according to a conventional method and packed in a hard capsule. Before packing, the granules may be coated as necessary with a conventional enteric coating (e.g., hydroxypropylmethylcellulose phthalate), sugar coating or film (e.g., ethylcellulose).

### Example 3: injection

| | |
|---|---|
| Compound 1 | 750 mg |
| carboxymethylcellulose sodium | 500 mg |
| water for injection | suitable amount |
| total amount | 100 mL |

The above components were aseptically admixed according to a conventional method to give an injection.

### Example 4: eye drop

| | |
|---|---|
| Compound 2 | 50 mg |
| boric acid | 700 mg |
| borax | suitable amount (pH 7.0) |
| sodium chloride | 500 mg |
| hydroxymethylcellulose | 0.5 g |
| sodium edetate | 0.05 mg |
| benzalkonium chloride | 0.005 mg |
| sterilized purified water | suitable amount |
| total amount | 100 mL |

Sterilized purified water (80 mL) was heated to about 80°C, hydroxymethylcellulose was added and the mixture is stirred until the liquid temperature reaches room temperature. Compound 2, sodium chloride, boric acid, sodium edetate and benzalkonium chloride are added to this solution to allow dissolution. A suitable amount of borax is added to adjust the pH to 7. Sterilized purified water is added to measure up to 100 mL.

### Example 5: eye drop

| | |
|---|---|
| acetic octoreotide | 112 mg |
| D-mannitol | 4.5 g |
| sodium dihydrogen phosphate | 0.1 g |
| sodium hydroxide | suitable amount (pH 7.0) |
| sterilized purified water | suitable amount |
| total amount | 100 mL |

Acetic octoreotide, D-mannitol and sodium dihydrogen phosphate are added to sterilized purified water (80 mL) to allow dissolution. A suitable amount of sodium hydroxide is added to adjust the pH to 5.0. Sterilized purified water is added to measure up to 100 mL. The prepared eye drop is aseptically filtered with a membrane filter and filled in a disposable (unit dose) container and sealed.

### Industrial Applicability

Since the pharmaceutical agent of the present invention, which contains a somatostatin receptor agonist, has a trigeminal nerve cell axon extension promoting action and an action to functionally recover decreased corneal sensitivity, it is useful for improving functional decrease of corneal sensitivity associated with corneal nerve damage and the like, and the symptoms of dry eye associated with functional decrease of corneal sensitivity. Specifically, application of a somatostatin receptor agonist is expected to provide an improvement effect on decreased corneal sensitivity after cataract surgery or LASIK surgery, decreased corneal sensitivity and dry eye associated with corneal neurodegeneration such as neuroparalytic keratopathy, corneal ulcer, diabetic keratopathy and the like.

While some of the embodiments of the present invention have been described in detail in the above, it will, however, be evident for those of ordinary skill in the art that various modifications and changes may be made to the particular embodiments shown without substantially departing from the novel teaching and advantages of the present invention. Such modifications and changes are encompassed in the spirit and scope of the present invention as set forth in the appended claims.

This application is based on a patent application Nos. 318881/2002 and 040250/2003 filed in Japan, the contents of which are hereby incorporated by reference.

## Claims

1. A corneal nerve axon extension promoter comprising a somatostatin receptor agonist.

2. An agent for the recovery of corneal sensitivity, which comprises a somatostatin receptor agonist.

3. A therapeutic agent for dry eye, which comprises a somatostatin receptor agonist.

4. A therapeutic agent for corneal epithelial defect, which comprises a somatostatin receptor agonist.

5. A pharmaceutical composition for promoting extension of corneal nerve axon, which comprises a somatostatin receptor agonist.

6. A pharmaceutical composition for the recovery of corneal sensitivity, which comprises a somatostatin receptor agonist.

7. A pharmaceutical composition for the treatment of dry eye, which comprises a somatostatin receptor agonist.

8. A pharmaceutical composition for the treatment of corneal epithelial defect, which comprises a somatostatin receptor agonist.

9. Use of a somatostatin receptor agonist for the production of a pharmaceutical composition for promoting extension of corneal nerve axon.

10. Use of a somatostatin receptor agonist for the production of a pharmaceutical composition for the recovery of corneal sensitivity.

11. Use of a somatostatin receptor agonist for the production of a pharmaceutical composition for the treatment of dry eye.

12. Use of a somatostatin receptor agonist for the production of a pharmaceutical composition for the treatment of corneal epithelial defect.

13. A method of promoting extension of corneal nerve axon, which comprises administering an effective amount of a somatostatin receptor agonist to a case in need of the promotion of extension of the corneal nerve axon.

14. A method of recovering corneal sensitivity, which comprises administering an effective amount of a somatostatin receptor agonist to a case in need of the recovery of corneal sensitivity.

15. A method of treating dry eye, which comprises administering an effective amount of a somatostatin receptor agonist to a case affected with dry eye.

16. A method of treating corneal epithelium defect, which comprises administering an effective amount of a somatostatin receptor agonist to a case having corneal epithelial defect.


•@                              Sequence Listing

<110> Senju Pharmaceutical Co. Ltd.
<120> A medicine of the corneal disorder
<130> 226-PCT

<160> 2

<210> 1
<211> 41
<212> DNA
<213> Artificial Sequence
<400> TGGCCGTCTT CATTTTCTGC TCGCCGCTCA CTTTGACCAA G•@ 41

<210> 2
<211> 42
<212> DNA
<213> Artificial Sequence
<400> GTGGGCAAGA TGCGCGCTGT GAATGGGGTT GGCGCAGCTG TT•@•@42